(19) Europäisches Patentamt
European Patent Office
Office européen des brevets

(11) Veröffentlichungsnummer: 0 012 117
B1

(12) **EUROPÄISCHE PATENTSCHRIFT**

(45) Veröffentlichungstag der Patentschrift: **10.02.82**

(51) Int. Cl.³: **C 07 D 213/61**, C 07 C 121/34

(21) Anmeldenummer: **79810171.3**

(22) Anmeldetag: **03.12.79**

(54) **Verfahren zur Herstellung von 2,3,5-Trichlorpyridin, 2,4,4-Trichlor-4-formyl-butyronitril als neue Verbindung und Verfahren zu dessen Herstellung.**

(30) Priorität: **05.12.78 CH 12394/78**
**05.12.78 CH 12395/78**
**23.10.79 CH 9488/79**
**23.10.79 CH 9489/79**

(43) Veröffentlichungstag der Anmeldung: **11.06.80 Patentblatt 80/12**

(45) Bekanntmachung des Hinweises auf die Patenterteilung: **10.02.82 Patentblatt 82/6**

(84) Benannte Vertragsstaaten: **AT BE CH DE FR GB IT LU NL SE**

(56) Entgegenhaltungen:
GB-A-1 334 922
US-A-3 592 817

(73) Patentinhaber: **CIBA-GEIGY AG, Patentabteilung Postfach, CH-4002 Basel (CH)**

(72) Erfinder: **Steiner, Eginhard, Dr., Obere Hofackerstrasse 3, CH-4414 Füllinsdorf (CH)**
Erfinder: **Martin, Pierre, Dr., Meisenweg 38, CH-4310 Rheinfelden (CH)**
Erfinder: **Bellus, Daniel, Dr., Unterm Schellenberg 81, CH-4125 Riehen (CH)**

EP 0 012 117 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2,3,5-Trichlorpyridin, 2,4,4-Trichlor-4-formylbutyronitril als neue Verbindung und Verfahren zu dessen Herstellung

Die vorliegende Erfindung betrifft ein Verfahren zur Herstellung von 2,3,5-Trichlorpyridin, 2,4,4-Trichlor-4-formylbutyronitril als neue Verbindung sowie ein Verfahren zu dessen Herstellung.

Die bisher bekanntgewordenen Verfahren zur Herstellung von 2,3,5-Trichlorpyridin sind in verschiedener Hinsicht unbefriedigend. 2,3,5-Trichlorpyridin kann z.B. durch mehrwöchiges Einwirken von Chlor auf mit Chlorwasserstoff gesättigtes Pyridin, durch längeres Erhitzen von Pyridin mit Phosphorpentachlorid auf 210 bis 220°C oder durch Erhitzen eines entwässerten Bariumsalzes der Pyridin-3,5-disulfonsäure mit Phosphorpentachlorid auf etwa 200°C erhalten werden. Dabei entstehen jedoch neben dem gewünschten 2,3,5-Trichlorpyridin auch beträchtliche Mengen anderer Chlorpyridine, vor allem Dichlorpyridine und Pentachlorpyridine, [vgl. „J. Chem. Soc.", *73*, 437 (1898), „J, Chem, Soc.", *93*, 2001 (1908) und „Ber. Dtsch. Chem. Ges.", *17*, 1832 (1884)].

2,3,5-Trichlorpyridin lässt sich auch durch Behandeln von 2-Amino-3,5-dichlorpyridin mit Kaliumnitrit in Gegenwart von Halogenwasserstoff, insbesondere konzentrierte Salzsäure, [vgl. Zentralblatt II, 1671 (1928) und britische Patentschrift Nr. 1215387] bzw. durch Erhitzen von 1-Methyl-2,3-dichlorpyrid-2-on mit Phosphorpentachlorid und ein wenig Phosphoroxichlorid bzw. mit Phosgen bei Temperaturen zwischen etwa 150 und 180°C herstellen [vgl. „J. pr. Chem.", (2), *93*, 371 (1916) und „Ann. Chem.", *486*, 71 (1931)]. Die für diese Synthesen benötigten Ausgangsprodukte sind nur durch mehrstufige und daher unwirtschaftliche Verfahren zugänglich; [vgl. z.B. „J. Org. Chem.", *23*, 1614 (1958), „Ber. dtsch. Chem. Ges.", *31*, 609 (1898) und ibid *32*, 1297 (1899)]. Auch vom ökologischen Standpunkt sind diese vorbekannten Verfahren wegen der in grossem Überschuss benötigten Chlorierungsmittel und anderer Hilfschemikalien nicht unbedenklich. Schliesslich sind auch die Ausbeuten an 2,3,5-Trichlorpyridin zum Teil unbefriedigend.

Anderseits ist aus der britischen Patentschrift Nr. 1024399 bekannt, dass sich Halogenverbindungen, wie Sulfonylhalogenide, Allylhalogenide und Halogennitrile in Gegenwart von Katalysatoren an äthylenisch ungesättigte Verbindungen, wie Olefine mit konjugierten Doppelbindungen, Acrylsäure und Acrylsäurederivate anlagern lassen. Dabei entstehen ausschliesslich offenkettige Produkte.

Schliesslich ist in der deutschen Offenlegungschrift Nr. 2709108 ein Verfahren zur Herstellung von 3,5-Dichlor-2-hydroxypyridinderivaten beschrieben. Nach diesem Verfahren lässt man Trichloracetonitril mit Alkenylaldehyden, z.B. Acrolein, oder Alkylalkenylketonen, z.B. Methylvinylketon, unter Zusatz von Radikalinitiatoren miteinander reagieren. Das dabei gebildete 2,2,4-Trichlorpentan-5-on-carbonsäurenitrilderivat kann thermisch oder durch Einwirkung von Lewis-Säuren cyclisiert und anschliessend durch Abspaltung von Chlorwasserstoff in ein 3,5-Dichlor-2-hydroxypyridinderivat umgewandelt werden.

Es wurde nun gefunden, dass man 2,3,5-Trichlorpyridin auf sehr einfache, wirtschaftliche und umweltfreundliche Weise in guten Ausbeuten und unter Verwendung von leicht zugänglichen, billigen Ausgangsmaterialien in der Weise herstellen kann, dass man Trichloracetaldehyd in Gegenwart eines Katalysators an Acrylnitril anlagert und das gebildete 2,4,4-Trichlor-4-formylbutyronitril der Formel I

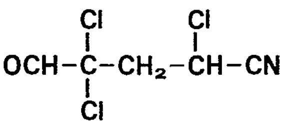 (I)

unter Wasserabspaltung zu 2,3,5-Trichlorpyridin cyclisiert.

Die Anlagerung von Trichloracetaldehyd an Acrylnitril kann in offenem oder geschlossenem System bei einer Temperatur von 70 bis 140°C durchgeführt werden. Bevorzugt erfolgt die Anlagerung in geschlossenem System bei einem der angewendeten Reaktionstemperatur entsprechendem Druck, der beispielsweise im Bereich von 1 bis 30 bar liegen kann.

Als Katalysatoren für die Anlagerung des Trichloracetaldehyds an das Acrylnitril können erfindungsgemäss Metalle der Hauptgruppe VIII und der Nebengruppen VIa, VIIa, Ib und IIb des periodischen Systems, z.B. Eisen, Kobalt, Nickel, Ruthenium, Rhodium, Palladium, Chrom, Molybdän, Mangan, Kupfer und Zink verwendet werden. Diese Metalle könen in elementarer Form oder in Form von Verbindungen eingesetzt werden. Geeignete Verbindungen sind beispielsweise Oxide, und Salze, wie Halogenide, Sulfate, Sulfite, Sulfide, Nitrate, Acetate, Stearate, Citrate, Carbonate, Cyanide und Rhodanide, sowie Komplexe mit Liganden, wie Phosphinen, Phosphiten, Benzoyl- und Acetylacetonaten, Nitrilen, Isonitrilen und Kohlenmonoxid.

Als Beispiele seien genannt: Kupfer(II)oxid, Eisen(III)oxid; Kupfer(I)-, Kupfer(II)-, Eisen(II)- und Eisen(III)bromide, -jodide und vor allem -chloride, Zinkchlorid, sowie die Chloride des Rutheniums, Rhodiums, Palladiums, Kobalts und Nickels; Kupfer(II)sulfat, Eisen(II)- und Eisen(III)sulfat; Kupfer(II) nitrat und Eisen(III)nitrat; Mangan(III)acetat, Kupfer(II)acetat, Kupfer(II) stearat, Eisen(III)citrat, Kupfer-(I)cyanid; Ruthenium(II)dichlorotristriphenyl-phosphin, Rhodiumdichlorotristriphenylphosphin; Chrom- und Nickelacetylacetonat, Kupfer(II)acetylacetonat, Eisen(III)acetylacetonat, Kobalt(II)- und Kobalt (III)acetylacetonat, Mangan(II)acetonylacetonat, Kupfer(II)benzylacetonat; Eisencarbonylcyclopentadienylkomplex; Molybdäncarbonylcyclopentadienylkomplex, Chromtricarbonylarylkomplexe, Ruthenium(II)acetato-komplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverebindung; Gemische von Kupferpulver mit Lithiumhalogeniden, wie Lithiumchlorid, oder Isocyaniden, wie tert.-Butylisocyanid; Gemische von Eisenpulver mit Eisen(III)chlorid, gegebenenfalls unter Zusatz von Kohlenmonoxid; Gemische von eisen(III)chlorid mit Benzoin; Gemische von Eisen(II)- oder Eisen(III)chlorid mit Trialkylphosphiten; Gemische von Eisenpentacarbonyl und Jod.

Bevorzugt sind Eisen(II)- und Eisen(III)salze und -komplexe, vor allem Eisen(II)- und Eisen(III)chlorid, sowie Eisenpulver; Ruthenium(III)-chlorid, Ruthenium(II)dichlorotristriphenylphosphin, Kupferbronze, Kupfer(I)- und Kupfer-(II)-salze und -komplexe, wie Kupfer(I)chlorid, Kupfer(II)chlorid, Kupfer(I)bromid, Kupfer(II)-bromid; Kupfer(II)acetat, Kupfer(II)acetyl-acetonat, Kupfer(II)benzoylacetonat, Kupfer(II)sulfat, Kupfer(II)nitrat, Kupfer(I)cyanid, und Kupfer(I)jodid.

Ganz besonders bevorzugt sind Kupferpulver, Kupferbronze, Kupfer(I)- und Kupfer(II)chlorid bzw. -bromid und Kupfer(I)jodid, sowie deren Gemische.

Die Katalysatoren werden im allgemeinen in Mengen von etwa 0,01 bis 10 mol%, bevorzugt 0,1 bis 5 mol%, bezogen auf das Acrylnitril, verwendet.

Die Anlagerung des Trichloracetaldehyds an das Acrylnitril wird zweckmässig in Gegenwart eines inerten organischen Lösungsmittels vorgenommen, Geeignete Lösungsmittel sind solche, in den die Katalysatoren ausreichend löslich sind oder die mit den Katalysatoren Komplexe bilden können, die aber gegenüber dem Trichloracetaldehyd und dem Acrylnitril inert sind. Als Beispiele für geeignete Lösungsmittel seien genannt: Alkancarbonsäurenitrile, insbesondere solche mit 2-5 Kohlenstoffatomen, wie Acetonitril, Propionitril und Butyronitril; 3-Alkoxypropionitrile mit 1-2 Kohlenstoffatomen in der Alkoxygruppe, wie 3-Methoxypropionitril und 3-Äthoxypropionitril; aromatische Nitrile, vor allem Benzonitril; aliphatische Ketone mit vorzugsweise insgesamt 3-8 Kohlenstoffatomen, wie Aceton, Diäthylketon, Methylisopropylketon, Diisopropylketon, Methyltert-butylketon; Alkyl- und Alkoxyalkylester von aliphatischen Monocarbonsäuren mit insgesamt 2-6 Kohlenstoffatomen, wie Ameisensäuremethyl- und -äthylester, Essigsäuremethyl-, äthyl-, -n-butyl- und -isobutylester, sowie 1-Acetoxy-2-methoxyäthan; cyclische Äther, wie Tetrahydrofuran, Tetrahydropyran und Dioxan; Dialkyläther mit je 1-4 Kohlenstoffatomen in den Alkylgruppen, wie Diäthyläther, Di-n-propyläther und Disopropyläther; N,N-Dialkylamide von Alkancarbonsäuren mit 1-3 Kohlenstoffatomen in der Alkylgruppe wie N,N-Dimethylformamid, N,N-Dimethylacetamid, N,N-Diäthylacetamid und N,N-Dimethylmethoxyacetamid; Äthylenglykol- und Diäthylenglykoldialkyläther mit je 1-4 Kohlenstoffatomen in den Alkylgruppen, wie Äthylenglykoldimethyl-, -diäthyl- und -di-n-butyläther; Diäthylenglykoldiethyl-, und -di-n-butyläther; Phosphorsäuretris-N,N-dimethylamid (Hexametapol). Ferner kann überschüssiges Acrylnitril als Lösungsmittel verwendet werden.

Bevorzugte Lösungsmittel für die Anlagerung von Trichloracetaldehyd an Acrylnitril sind Alkancarbonsäurenitrile mit 2-3 Kohlenstoffatomen und 3-Alkoxypropionitrile mit 1-2 Kohlenstoffatomen in der Alkoxygruppe, insbesondere Acetonitril, Butyronitril, Acrylnitril und 3-Methoxypropionitril.

Das durch Anlagerung von Trichloracetaldehyd an Acrylnitril herstellbare 2,4,4-Trichlor-4-formylbutyronitril ist eine neue Verbindung und ebenfalls Gegenstand der Erfindung.

Die Cyclisierung des 2,4,4-Trichlor-4-formylbutyronitrils kann in einem offenen oder einem geschlossenen Systems bei Temperaturen zwischen etwa 0 und 220°C, insbesondere zwischen etwa 80 und 200°C durchgeführt werden. Vorzugsweise wird die Cyclisierung in einem offenen System durchgeführt. Bei der Cyclisierung in einem offenen System ist es vorteilhaft, diese in Gegenwart von Chlorwasserstoff oder in Gegenwart von Substanzen, welche unter Reaktionsbedingungen Chlorwasserstoff bilden, wie Phosgen, Bortrichlorid, Aluminiumchlorid, Trialkylammoniumchloride mit je 1-4 Kohlenstoffatomen in den Alkylgruppen, Phosphorpentachlorid, Phosphoroxychlorid oder Phosphortrichlorid, vorzunehmen.

Vorzugsweise wird die Cyclisierung in Gegenwart von Bromwasserstoff und insbesondere Chlorwasserstoff vorgenommen.

Die Cyclisierung kann entweder ohne Zusatz eines Lösungsmittels, in der Flüssig- oder in der Gasphase durch blosses Erhitzen des 2,4,4-Trichlor-4-formylbutyronitrils oder aber in Gegenwart eines organischen Lösungsmittels durchgeführt werden. Als organische Lösungsmittel eignen sich beispielsweise chlorierte aliphatische Kohlenwasserstoffe, wie Chloroform, Methylenchlorid und Tetrachloräthan; gegebenenfalls chlorierte aromatische Kohlenwasserstoffe, wie Benzol, Toluol, Xylole und Chlorbenzole; N,N-Dialkylamide von Alkancarbonsäuren mit 1-3 Kohlenstoffatomen, wie N,N-Dimethylformamid, N,N-Dimethylacidamid, N,N-Diäthylacetamid und N,N-Dimethylmethoxyacetamid; cyclische Amide, wie N-Methyl-2-pyrrolidon, N-Acetyl-2-pyrrolidon und N-Methyl-ε-caprolactam; Amide der Kohlensäure, wie Tetramethylharnstoff und Dimorpholinocarbonyl; Amide der phosphorigen Säure, der Phosphorsäure, der Phenylphosphonsäure oder von Alkylphosphonsäuren mit 1-3 Kohlenstoffatomen in der Alkylgruppe, wie Phosphorsäuretriamid, Phosphorsäuretris-(N,N-dimethylamid), Phosphorsäuretrimorpholid, Phosphorsäuretripyrrolinid, Phosphorigsäuretris-(N,N-dimethylamid), Methanphosphonsäurebis-(N,N-dimethylamid); Amide der Schwefelsäure oder

von aliphatischen oder aromatischen Sulfonsäuren, wie Tetramethylsulfamid, Methansulfonsäuredimethylamid, oder p-Toluolsulfonsäureamid; aliphatische Ketone, cyclische Äther, Dialkyläther sowie Äthylenglykol- und Diäthylenglykoldialkyläther der vorerwähnten Art, sowie Phosphortrichlorid und Phosphoroxychlorid.

Bevorzugte Lösungsmittel für die Cyclisierung sind Chloroform, Methylenchlorid, cyclische Äther und Dialkyläther mit je 1-4 Kohlenstoffatomen in den Alkylgruppen, insbesondere Dioxan und Diäthyläther, sowie N,N-Dialkylamide von niederen aliphatischen Carbonsäuren, insbesondere N,N-Dimethylformamid.

Das erfindungsgemässe Verfahren kann in der Weise durchgeführt werden, dass man das durch Anlagerung von Trichloracetaldehyd an Acrylnitril gebildete 2,4,4-Trichlor-4-formylbutyronitril zunächst isoliert und anschliessend in einer zweiten Verfahrensstufe cyclisiert. Dabei werden die einzelnen Verfahrensstufen wie vorstehend beschrieben durchgeführt.

Gemäss einer vorteilhaften Ausführungsform des erfindungsgemässen Verfahrens wird Trichloracetaldehyd bei einer Temperatur von 70 bis 140°C in einem inerten Lösungsmittel in Gegenwart von 0,1 bis 5 mol% Kupferpulver, Kupferbronze, Kupfer(I)- und Kupfer(II)chlorid bzw. -bromid oder Kupfer(I)jodid oder in Gegenwart eines Gemisches dieser Substanzen in einem geschlossenen System mit Acrylnitril umsetzt und das nach Abtrennung des Lösungsmittels erhaltene 2,4,4-Trichlor-4-formylbutyronitril bei einer Temperatur zwischen 80 und 200°C in einem offenen System in Gegenwart von Chlorwasserstoff oder einer Substanz, welche bei Reaktionsbedingungen Chlorwasserstoff bildet, zu 2,3,5-Trichlorpyridin cyclisiert.

Man kann jedoch auch auf die Isolierung des 2,4,4-Trichlor-4-formylbutyronitrils verzichten und die Additions- und die Cyclisierungsreaktion in einem Arbeitsgang durchführen. In diesem Fall erfolgt die Umsetzung von Trichloracetaldehyd und Acrylnitril zum 2,3,5-Trichlorpyridin bei einer Temperatur zwischen etwa 70 und 220°C, insbesondere zwischen etwa 130 und 200°C. Dabei kann in offenem oder geschlossenem System gearbeitet werden. Wird die Umsetzung in offenem System durchgeführt, so kann es zweckmässig sein, diese in Gegenwart von Chlorwasserstoff oder in Gegenwart von Substanzen vorzunehmen, welche unter Reaktionsbedingungen Chlorwasserstoff bilden. Derartige Substanzen sind beispielsweise Phosgen, Bortrichlorid, Aluminiumchlorid, Trialkylammoniumchloride mit 1-4 Kohlenstoffatomen in den Alkylgruppen, Phosphorpentachlorid, Phosphoroxychlorid oder Phosphortrichlorid. Die einstufige Herstellung von 2,3,5-Trichlorpyridin erfolgt vorzugsweise in einem geschlossenen System bei einem der jeweiligen Reaktionstemperatur entsprechenden Druck, der beispielsweise je nach Reaktionstemperatur im Bereich von 1 bis 50 bar liegen kann. Besonders bevorzugt ist die Herstellung des 2,3,5-Trichlorpyridin in einem geschlossenen System bei einem Druck von 1 bis 30 bar.

Bevorzugte Lösungsmittel für die einstufige Durchführung des Verfahrens sind Alkancarbonsäurenitrile mit 2-5 Kohlenstoffatomen und 3-Alkoxypropionitrile mit 1-2 Kohlenstoffatomen in der Alkylgruppe. Besonders geeignete Lösungsmittel sind Acetonitril, Butyronitril und 3-Methoxypropionitril. Nach Beendigung der Reaktion kann das 2,3,5-Trichlorpyridin auf übliche Weise, z.B. durch Abdampfen des Lösungsmittels und Reinigung des Rohprodukts durch Wasserdampfdestillation isoliert werden.

Gemäss einer weiteren vorteilhaften Ausführungsform des erfindungsgemässen Verfahrens setzt man Trichloracetaldehyd und Acrylnitril in Acetonitril, Butyronitril oder 3-Methoxypropionitril als Lösungsmittel in Gegenwart von 0,1 bis 5 mol% Kupferpulver, Kupferbronze, Kupfer(I)- und Kupfer(II)chlorid bzw. -bromid oder Kupfer(I)-jodid oder einem Gemisch dieser Substanzen bei 130 bis 200°C in einem geschlossenen System bei einem der jeweils angewandten Reaktionstemperatur entsprechenden Druck direkt zu 2,3,5-Trichlorpyridin um.

Mit dem erfindungsgemässen Verfahren wird es möglich, 2,3,5-Trichlorpyridin auf sehr einfache, wirtschaftliche und umweltfreundliche Weise in guten Ausbeuten und unter Verwendung von leicht zugänglichen, billigen Ausgangsmaterialien herzustellen. Dabei ist es überraschend, dass bei Verwendung der erfindungsgemässen Ausgangsmaterialien ein Additionsprodukt erhalten wird, das sich im Gegensatz zu vorbekannten, strukturell ähnlichen Additionsprodukten unter Verschiebung eines Chloratoms leicht zum 2,3,5-Trichlorpyridin cyclisieren lässt. Es wird also bei Verwendung eines Ausgangsproduktes (Trichloracetaldehyd), in welchem alle Chloratome an dasselbe Kohlenstoffatom gebunden sind, ein Endprodukt erhalten, in dem sich die drei Chloratome an verschiedenen Kohlenstoffatomen und in den gewünschten Stellungen befinden. Dank dieser völlig unerwarteten Reaktion kann auf die Verwendung von Chlorierungsmitteln und weiterer Hilfsreagenzien verzichtet werden.

Das 2,3,5-Trichlorpyridin kann auf an sich bekannte Weise zur Herstellung verschiedener Wirkstoffe, besonders von Herbiziden, verwendet werden (vgl. z.B. US Patentschriften Nr. 3814774, 3894862 und 4046553).

Das erfindungsgemässe Verfahren wird durch die folgenden Beispiele näher erläutert.

*Beispiel 1:*

a) Herstellung von 2,4,4-Trichlor-4-formylbutyronitril.

22,0 g Trichloracetaldehyd, 5,3 g Acrylnitril und 0,5 g Kupfer(I)chlorid werden mit 30 ml Acetonitril in einem Emailleautoklaven während 20 h auf 115°C erhitzt. Nach dem Erkalten wird das Lösungsmittel am Wasserstrahlvakuum bei ca. 40 bis 50°C abdestilliert, der Rückstand wird mit 50 ml Diäthyläther versetzt, und das ausgefallene Kupfer(I)chlorid wird abfiltriert. Nach dem Abdestillieren des Diäthyläthers wird der Rückstand im Hochvakuum rektifiziert, und die bei 64 bis 65°C und 400 Pa siedende Fraktion wird abgefangen. Man erhält 13,6 g (60% d.Th.) 2,4,4-Trichlor-4-formylbutyronitril in Form eines farblosen Öls.

IR-Spektrum ($CHCl_3$) in $cm^{-1}$: 2250 (CN), 1750(CO).

1H-NMR-Spektrum (60 MHz in $CDCl_3$) in ppm: 9,15 (s, 1H, –CHO); 4,85 (t, 1H, H an C-2); 3,1 (d, 2H, 2H an C-3).

Elementaranalyse für $C_5H_4Cl_3NO$ (Molgewicht 200,45):

| | | | | |
|---|---|---|---|---|
| berechnet | C 29,96 | H 2,01 | N 6,99 | Cl 53,06% |
| gefunden | C 29,89 | H 2,13 | N 6,95 | Cl 52.67% |

b) Herstellung des 2,3,5-Trichlorpyridins.

13,6 g des gemäss a) erhaltenen 2,4,4-Trichlor-4-formylbutyronitrils werden unter Zusatz von 1,0 g $AlCl_3$ in einem Emailleautoklaven 1 h auf 60°C erhitzt. Danach wird das dunkle Rohprodukt mit Wasserdampf destilliert, wobei sich das 2,3,5-Trichlorpyridin im Destillat in Form von weissen Kristallen abscheidet. Die Ausbeute beträgt 9,1 g (83% d. Th.) 2,3,5-Trichlorpyridin vom Fp. 49 bis 50°C.

*Beispiel 2:*

a) 14,7 g Trichloracetaldehyd, 5,3 g Acrylnitril und 0,5 g. Kupfer(I)chlorid werden in 40 ml 3-Methoxypropionitril auf 80 bis 85°C erhitzt. Im Verlaufe der Reaktion steigt der Siedepunkt des Gemisches an und erreicht nach ca. 30 h 125 bis 130°C. Nach dem Erkalten wird der dunkle Kolbeninhalt mit Diälthyläther extrahiert. Nach dem Abdestillieren des Diäthyläthers wird das zurückbleibende braune Öl im Wasserstrahlvakuum auf 100°C (Badtemperatur) erhitzt, wobei das 3-Methoxypropionitril abdestilliert. Der Rückstand wird im Hochvakuum rektifiziert. Man erhält 10,2 g (51% d. Th.) eines farblosen Öls, das mit dem gemäss Beispiel 1a) erhaltenen Produkt identisch ist.

b) Das gemäss dem obigen Abschnitt a) erhaltene 2,4,4-Trichlor-4-formylbutyronitril wird durch 3stündiges Erhitzen auf 130°C und anschliessende Wasserdampfdestillation in das 2,3,5-Trichlorpyridin übergeführt (Ausbeute 80% d. Th.).

*Beispiel 3:*

20 g des nach Beispiel 1a) hergestellten 2,4,4-Trichlor-4-formylbutyronitrils werden in 20 ml Diälthyläther gelöst und bei 20 bis 25°C während 5 h mit einem Strom von Bromwasserstoffgas behandelt. Anschliessend wird der Diäthyläther im Vakuum abdestilliert, und der Rückstand wird durch Wasserdampfdestillation gereinigt. Man erhält 13,6 g (60% d. Th.) 2-Brom-3,5-dichlorpyridin in Form weisser Kristalle vom Fp. 42°C.

Eine ähnlich gute Ausbeute wird erhalten, wenn bei sonst gleicher Arbeitsweise statt Diäthyläther Chloroform als Lösungsmittel verwendet wird.

*Beispiel 4:*

a) Herstellung von 2,4,4-Trichlor-4-formylbutyronitril.

14,7 g Trichloracetaldehyd, 13,2 g Acrylnitril und 0,63 g Kupferpulver (aktiviert nach dem in „Org. Synth.", Coll. Vol. III, 339 für Kupferbronze angegebenen Verfahren) werden in einem Druckreaktor während 12 h auf 105°C erhitzt. Anschliessend wird das überschüssige Acrylnitril bei 40 bis 50°C im Wasserstrahlvakuum abdestilliert. Als Rückstand werden 18,2 g eines dunklen Öls erhalten, das nach gaschromatographischer Analyse zu 85,4% aus 2,4,4-Trichlor-4-formylbutyronitril besteht, was einer Ausbeute von 77% d. Th. entspricht.

b) Herstellung von 2,3,5-Trichlorpyridin.

Das nach a) erhaltene 2,4,4-Trichlor-4-formylbutyronitril (18,2 g) wird innerhalb von 15 min in ein 40 cm langes und 2,5 cm weites, zur Hälfte mit Raschig-Ringen gefülltes, vertikales Mantelrohr, dessen Mantel mit heissem Öl auf 175 bis 180°C erhitzt ist, eingetropft. Gleichzeitig wird von unten ein schwacher Strom von Chlorwasserstoff dem Reaktionsgemisch entgegengeleitet. Das aus dem Reaktor austropfende dunkle Harz wird mit Wasserdampf destilliert. Man erhält 11,5 g (85% d. Th.) 2,3,5-Trichlorpyridin in Form weisser Kristalle vom Fp. 49 bis 50°C.

*Beispiel 5:*

a) Herstellung von 2,4,4-Trichlor-4-formylbutyronitril.

Ein Gemisch aus 14,7 g Trichloracetaldehyd, 13,2 g Acrylnitril und 0,63 g Kupferbronze (aktiviert nach dem in „Org. Synth.", Coll. Vol. III, 339 angegebenen Verfahren) wird 48 h unter Rückfluss erhitzt. Anschliessend wird das überschüssige Acrylnitril bei 40 bis 50°C im Wasserstrahlvakuum abdestilliert. Als Rückstand werden 17,3 g eines dunklen Öls erhalten, das nach gaschromatographischer Analyse zu 88,5% aus 2,4,4-Trichlor-4-formylbutyronitril besteht, was einer Ausbeute von 76,5% d. Th. entspricht.

b) Herstellung von 2,3,5-Trichlorpyridin.

Das nach a) erhaltene 2,4,4-Trichlor-4-formylbutyronitril (17,3 g) wird unter Einleiten eines schwachen Stromes von Chlorwasserstoff 24 h auf 80 bis 85°C erhitzt. Anschliessend wird das gesamte Reaktionsgemisch mit Wasserdampf destilliert. Man erhält 10,0 g (72% d. Th.) 2,3,5-Trichlorpyridin in Form weisser Kristalle vom Fp. 49 bis 50°C.

*Beispiel 6:*

Herstellung von 2,3,5-Trichlorpyridin.

In eine Lösung von 25,0 g (0,125 mol) 2,4,4-Trichlor-4-formylbutyronitril in 50 ml N,N-Dimethylformamid wird Chlorwasserstoff mit solcher Geschwindigkeit eingeleitet, dass die Temperatur des Reaktionsgemisches 120°C nicht übersteigt. Nach beendigter Umsetzung wird das Reaktionsgemisch auf Eiswasser gegossen. Der

beige Niederschlag wird abfiltriert und getrocknet. Man erhält so 15,1 g (66% d. Th.) 2,3,5-Trichlorpyridin vom Fp. 48 bis 50°C.

*Beispiel 7:*

Herstellung von 2,3,5-Trichlorpyridin.

10,3 g Phosphorpentachlorid werden unter Kühlung portionsweise bei maximal 60°C in 40,0 g Dimethylformamid eingetragen. Anschliessend wird die erhaltene Lösung mit Chlorwasserstoff gesättigt, wobei die Temperatur bis 95°C ansteigt. Nach Abkühlung auf 50°C werden 20,0 g 2,4,4-Trichlor-4-formylbutyronitril (hergestellt nach Beispiel 1a)) so zugetropft, dass die Temperatur von 75°C nicht überschritten wird. Nach beendigter Zugabe des 2,4,4-Trichlor-4-formylbutyronitrils wird die Mischung 1 h auf 100°C erhitzt. Anschliessend wird das Reaktionsgemisch bei 60°C auf Eis gegossen, wobei sich das 2,3,5-Trichlorpyridin in fester Form abscheidet. Nach Filtration und Trocknung werden 16,2 g (89% d. Th.) 2,3,5-Trichlorpyridin vom Fp. 49 bis 51°C erhalten.

*Beispiel 8:*

17,7 g Trichloracetaldehyd, 5,3 g Acrylnitril und 0,5 g Kupfer(I)chlorid werden mit 40 ml Acetonitril in einem Emailleautoklaven während 1 h auf 180°C erhitzt. Nach dem Erkalten wird das Lösungsmittel bei ca. 40 bis 50°C im Wassertrahlvakuum abdestilliert. Der Rückstand wird einer Wasserdampfdestillation unterworfen. Im Destillat fällt das 2,3,5-Trichlorpyridin in Form weisser Kristalle vom Fp. 49 bis 50°C aus. Ausbeute=11,1 g (61% d. Th.).

Das 2,3,5-Trichlorpyridin wird in ähnlich guter Ausbeute erhalten, wenn man anstatt Kupfer(I)-chlorid 0,5 g Kupferbronze (hergestellt nach „Organic Syntheses", Coll. Vol. III, 339) oder 0,5 g wasserfreies Eisen(II)chlorid verwendet und sonst wie oben beschrieben vorgeht.

*Beispiel 9:*

17,7 g Trichloracetaldehyd, 5,3 g Acrylnitril, 0,3g Ruthenium(II)dichlorotristriphenylphosphin [T.A. Stephenson und G. Wilkinson, „Inorg. Nucl. Chem.", *28*, 945 (1966)] und 30 ml 3-Methoxypropionitril werden in einem Emailleautoklaven während 2 h auf 170°C erhitzt. Nach der Aufarbeitung analog Beispiel 1 erhält man 10,5 g (58% d. Th.) 2,3,5-Trichlorpyridin.

Das 2,3,5-Trichlorpyridin wird in ebenfalls guter Ausbeute erhalten, wenn man im obigen Beispiel das 3-Methoxypropionitril durch Butyronitril ersetzt.

*Beispiel 10:*

17,7 g Trichloracetaldehyd, 5,3 g Acrylnitril und 0,5 g Kupfer(I)chlorid werden mit 40 ml Acetonitril in einem Emailleautoklaven während ½ h auf 190°C erhitzt. Nach dem Erkalten wird das Lösungsmittel bei ca. 40 bis 50°C im Wasserstrahlvakuum abdestilliert. Der Rückstand wird einer Wasserdampfdestillation unterworfen. Im Destillat fällt das 2,3,5-Trichlorpyridin in Form weisser Kristalle vom Fp. 49 bis 50°C aus. Ausbeute=11,3 g (62% d. Th.).

Das 2,3,5-Trichlorpyridin wird in ähnlich guter Ausbeute erhalten, wenn bei sonst gleicher Arbeitsweise die Reaktionstemperatur während 2 h auf 170°C oder 6 h auf 150°C gehalten wird.

**Patentansprüche**

1. Verfahren zur Herstellung von 2,3,5-Trichlorpyridin, dadurch gekennzeichnet, dass man Trichloracetaldehyd in Gegenwart eines Katalysators an Acrylnitril anlagert und das gebildete 2,4,4-Trichlor-4-formylbutylnitril der Formel I

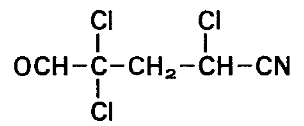

(I)

unter Wasserabspaltung zu 2,3,5-Trichlorpyridin cyclisiert.

2. Verfahren nach Anspruch 1, dadurch gekennzeichnet, dass man die Anlagerung von Trichloracetaldehyd an Acrylnitril bei einer Temperatur von 70 bis 140°C durchführt.

3. Verfahren nach Anspruch 1, dadurch gekennzeichnet, dass man die Anlagerung von Trichloracetaldehyd an Acrylnitril in einem geschlossenen System bei einer Temperatur von 70 bis 140°C und einem der angewendeten Reaktionstemperatur entsprechenden Druck durchführt.

4. Verfahren nach Anspruch 1, dadurch gekennzeichnet, dass man als Katalysator für die Anlagerung des Trichloracetaldehyds an das Acrylnitril ein Metall der Hauptgruppe VIII oder der Nebengruppen VIa, VIIa, Ib und IIb, ein Oxid eines solchen Metalls, ein Salz eines solchen Metalls oder eine Komplexverbindung eines solchen Metalls verwendet.

5. Verfahren nach Anspruch 1, dadurch gekennzeichnet, dass man als Katalysator für die Anlagerung von Trichloracetaldehyd an Acrylnitril Eisen(II)- und Eisen(III)salze und -komplexe, Eisenpulver, Ruthenium(III)chlorid, Ruthenium-(II)dichlorotristriphenylphosphin, Kupferpulver, Kupferbronze, Kupfer(I)- und Kupfer(II)salze und -komplexe verwendet.

6. Verfahren nach den Ansprüchen 1 und 5, dadurch gekennzeichnet, dass man als Katalysator für die Anlagerung von Trichloracetaldehyd an Acrylnitril Kupferpulver, Kupferbronze, Kupfer(I)- und Kupfer(II)chlorid bzw. -bromid, Kupfer(I) jodid, sowie deren Gemische verwendet.

7. Verfahren nach den Ansprüchen 1 und 4 bis 6, dadurch gekennzeichnet, dass man den Katalysator für die Anlagerung von Trichloracetaldehyd an Acrylnitril in Mengen von etwa 0,01 bis 10 mol%, vorzugsweise 0,1 bis 5 mol%, bezogen auf das Acrylnitril, verwendet.

8. Verfahren nach Anspruch 1, dadurch gekennzeichnet, dass man die Anlagerung von Trichloracetaldehyd an Acrylnitril in Gegenwart eines inerten organischen Lösungsmittels durchführt.

9. Verfahren nach den Ansprüchen 1 und 8, dadurch gekennzeichnet, dass man die Anlagerung von Trichloracetaldehyd an Acrylnitril in einem Alkancarbonsäurenitril mit 2-5 Kohlenstoffatomen, einem 3-Alkoxypropionitril mit 1-2 Kohlenstoffatomen in der Alkylgruppe oder in überschüssigem Acrylnitril als Lösungsmittel durchführt.

10. Verfahren nach Anspruch 1, dadurch gekennzeichnet, dass man die Cyclisierung des durch Anlagerung von Trichloracetaldehyd an Acrylnitril gebildeten 2,4,4-Trichlor-4-formylbutyronitrils bei einer Temperatur zwischen 0 und 220° C, vorzugsweise zwischen etwa 80 und 200° C durchführt.

11. Verfahren nach Anspruch 1, dadurch gekennzeichnet, dass man die Cyclisierung bei einer Temperatur zwischen 80 und 200° C in einem offenen System in Gegenwart von Chlorwasserstoff oder in Gegenwart von Substanzen, welche unter Reaktionsbedingungen Chlorwasserstoff bilden, durchführt.

12. Verfahren nach den Ansprüchen 1 und 11, dadurch gekennzeichnet, dass man die Cyclisierung des 2,4,4-Trichlor-4-butyronitrils in Gegenwart von Bromwasserstoff und insbesondere in Gegenwart von Chlorwasserstoff durchführt.

13. Verfahren nach Anspruch 1, dadurch gekennzeichnet, dass man die Cyclisierung des 2,4,4-Trichlor-4-formylbutyronitrils in Abwesenheit eines Lösungsmittels durch Erhitzen in der Flüssig- oder in der Gasphase vornimmt.

14. Verfahren nach Anspruch 1, dadurch gekennzeichnet, dass man die Cyclisierung des 2,4,4-Trichlor-4-formylbutyronitrils in Gegenwart eines organischen Lösungsmittels vornimmt.

15. Verfahren nach den Ansprüchen 1 und 14, dadurch gekennzeichnet, dass man die Cyclisierung des 2,4,4-Trichlor-4-formylbutyronitrils in Gegenwart von Chloroform, Methylenchlorid, cyclischen Äthern, Dialkyläthern mit 1-4 Kohlenstoffatomen in den Alkylgruppen oder N,N-Dialkylamiden von niederen aliphatischen Carbonsäuren durchführt.

16. Verfahren nach Anspruch 1, dadurch gekennzeichnet, dass man das durch Anlagerung von Trichloracetaldehyd an Acrylnitril gebildete 2,2,4-Trichlor-4-formylbutyronitril zunächst isoliert und anschliessend in einer zweiten Verfahrensstufe cyclisiert.

17. Verfahren nach den Ansprüchen 1 und 16, dadurch gekennzeichnet, dass man Trichloracetaldehyd bei einer Temperatur von 70 bis 140° C in einem inerten Lösungsmittel in Gegenwart von 0,1 bis 5 mol% Kupferpulver, Kupferbronze, Kupfer(I)- und Kupfer(II)chlorid bzw. -bromid oder Kupfer(I)jodid oder in Gegenwart eines Gemisches dieser Substanzen in einem geschlossenen System mit Acrylnitril umsetzt und das erhaltene 2,2,4-Trichlor-4-formylbutyronitril bei einer Temperatur zwischen 80 und 200° C in einem offenen System in Gegenwart von Chlorwasserstoff oder einer Substanz, welche bei Reaktionsbedingungen Chlorwasserstoff bildet, zu 2,3,5-Trichlorpyridin cyclisiert.

18. Verfahren nach Anspruch 1, dadurch gekennzeichnet, dass man Trichloracetaldehyd und Acrylnitril bei einer Temperatur zwischen 70 und 220° C in Gegenwart eines Katalysators ohne Isolierung des intermediär gebildeten 2,2,4-Trichlor-4-formylbutyronitrils direkt zum 2,3,5-Trichlorpyridin umsetzt.

19. Verfahren nach den Ansprüchen 1 und 18, dadurch gekennzeichnet, dass man die Umsetzung von Trichloracetaldehyd und Acrylnitril in einem geschlossenen System bei einem der jeweiligen Reaktionstemperatur entsprechenden Druck durchführt.

20. Verfahren nach den Ansprüchen 1, 18 und 19, dadurch gekennzeichnet, dass man die Umsetzung von Trichloracetaldehyd und Acrylnitril in Gegenwart eines Alkancarbonsäurenitrils mit 2-5 Kohlenstoffatomen oder eines 3-Alkoxypropionitrils mit 1-2 Kohlenstoffatomen in der Alkylgruppe als Lösungsmittel durchführt.

21. Verfahren nach den Ansprüchen 1 und 18 bis 20, dadurch gekennzeichnet, dass man Trichloracetaldehyd und Acrylnitril in Acetonitril, Butyronitril oder 3-Methoxypropionitril als Lösungsmittel in Gegenwart von 0,1 bis 5 mol% Kupferpulver, Kupferbronze, Kupfer(I)- oder Kupfer(II) chlorid bzw. -bromid oder Kupfer(I)jodid oder einem Gemisch dieser Substanzen bei 130 bis 200° C in einem geschlossenen System bei einem der jeweils angewendeten Reaktionstemperatur entsprechenden Druck direkt zu 2,3,5-Trichlorpyridin umsetzt.

22. 2,4,4-Trichlor-4-formylbutyronitril.

23. Verfahren zur Herstellung von 2,4,4-Trichlor-4-formylbutyronitril, dadurch gekennzeichnet, dass man Trichloracetaldehyd in Gegenwart eines Katalysators an Acrylnitril anlagert.

24. Verfahren nach Anspruch 23, dadurch gekennzeichnet, dass man die Anlagerung von Trichloracetaldehyd an Acrylnitril in Gegenwart eines organischen Lösungsmittels bei einer Temperatur von 70 bis 140° C vornimmt.

25. Verfahren nach den Ansprüchen 23 und 24, dadurch gekennzeichnet, dass man die Anlagerung von Trichloracetaldehyd an Acrylnitril in Gegenwart von überschüssigem Acrylnitril als Lösungsmittel durchführt.

26. Verfahren nach Anspruch 23, dadurch gekennzeichnet, dass man die Anlagerung von Trichloracetaldehyd an Acrylnitril in Gegenwart eines Metalls der Hauptgruppe VIII oder der Nebengruppen VIa, VIIa, Ib oder IIb des periodischen Systems, eines Oxids oder eines Salzes eines solchen Metalls als Katalysator durchführt.

27. Verfahren nach den Ansprüchen 23 und 26, dadurch gekennzeichnet, dass man als Katalysator für die Anlagerung von Trichloracetaldehyd an Acrylnitril Eisen(II)chlorid, Eisen(III)chlorid, Eisenpulver, Ruthenium(III)chlorid, Ruthenium(II)dichlorotristriphenylphosphin, Kupferpulver, Kupferbronze, Kupfer(I)chlorid, Kupfer(II)chlorid, Kupfer(I)bromid, Kupfer(II)bromid, Kupfer(II) acetat, Kupfer(II)acetylacetonat, Kupfer(II) benzylacetonat, Kupfer(II)sulfat, Kupfer(II)nitrat, Kupfer(I)cyanid oder Kupfer(I)jodid verwendet.

28. Verfahren nach den Ansprüchen 23 und 26, dadurch gekennzeichnet, dass man als Katalysator für die Anlagerung von Trichloracetaldehyd an Acrylnitril Kupferpulver, Kupferbronze, Kupfer(I) bromid oder Kupfer(I)jodid oder deren Gemische verwendet.

29. Verfahren nach den Ansprüchen 23 und 26 bis 28, dadurch gekennzeichnet, dass man die Katalysatoren für die Anlagerung von Trichloracetaldehyd an Acrylnitril in Mengen von 0,01 bis 10 mol% vorzugsweise 0,1 bis 5 mol%, bezogen auf Acrylnitril, verwendet.

**Claims:**

1. A process for producing 2,3,5-trichloropyridine, which process comprises causing trichloroacetaldehyde to undergo an addition reaction, in the presence of a catalyst, with acrylonitrile, and cyclising the formed 2,4,4-trichloro-4-formylbutyronitrile of the formula I

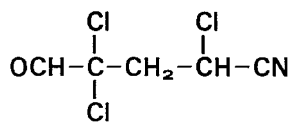

(I)

with the splitting-off of water, to give 2,3,5-trichloropyridine.

2. A process according to claim 1, wherein the addition reaction of trichloroacetaldehyde with acrylonitrile is performed at a temperature of 70 to 140°C.

3. A process according to claim 1, wherein the addition reaction of trichloroacetaldehyde with acrylonitrile is performed in a closed system at a temperature of 70 to 140°C and at a pressure corresponding to the applied reaction temperature.

4. A process according to claim 1, wherein there is, used as catalyst for the addition reaction of trichloroacetaldehyde with acrylonitrile, a metal of the main group VIII or of the subgroups VIa, VIIa, Ib and IIb, an oxide of a metal of this type, a salt of a metal of this type or a complex compound of a metal of this type.

5. A process according to claim 1, wherein the catalyst used for the addition reaction of trichloroacetaldehyde with acrylonitrile is selected from the group consisting of iron(II)- and iron(III)salts or complexes thereof, iron powder, ruthenium(III) chloride, ruthenium(II)dichlorotristriphenylphosphine, copper powder, copper bronze, copper(I)- or copper(II)salts or complexes thereof.

6. A process according to claims 1 and 5, wherein the catalyst used for the addition reaction of trichloroacetyldehyde with acrylonitrile is selected from the group consisting of copper powder, copper bronze, copper(I)- or copper(II)chloride or -bromide, copper(I)iodide, or mixtures thereof.

7. A process according to claim 1 and 4 to 6, wherein the catalyst for the addition reaction of trichloroacetaldehyde with acrylonitrile is used in amounts of about 0.01 to 10 mol%, preferably 0,1 to 5 mol%, relative to the acrylonitrile.

8. A process according to claim 1, wherein the addition reaction of trichloroacetaldehyde with acrylonitrile is performed in the presence of an inert organic solvent.

9. A process according to claims 1 and 8, wherein the addition reaction of trichloroacetaldehyde with acrylonitrile is performed in an alkanecarboxylic acid nitrile having 2 to 5 carbon atoms, in a 3-alkoxypropionitrile having 1 to 2 carbon atoms in the alkyl group, or in excess acrylonitrile as the solvent.

10. A process according to claim 1, wherein cyclisation of the 2,4,4-trichloro-4-formylbutyronitrile formed by the addition reaction of trichloroacetaldehyde with acrylonitrile is performed at a temperature of between 0 and 220°C, preferably between about 80 and 200°C.

11. A process according to claim 1, wherein the cyclisation reaction is performed at a temperature of between 80 and 200°C in an open system, and in the presence of hydrogen chloride or in the presence of substances which form hydrogen chloride under the reaction conditions.

12. A process according to claims 1 and 11, wherein cyclisation of 2,4,4-trichloro-4-butyronitrile is performed in the presence of hydrogen bromide, and particularly in the presence of hydrogen chloride.

13. A process according to claim 1, wherein cyclisation of 2,4,4-trichloro-4-formylbutyronitrile is performed, in the absence of a solvent, by heating in the liquid or in the gaseous phase.

14. A process according to claim 1, wherein cyclisation of 2,4,4-trichloro-4-formylbutyronitrile is performed in the presence of an organic solvent.

15. A process according to claims 1 and 14, wherein cyclisation of 2,4,4-trichloro-4-formylbutyronitrile is performed in the presence of chloroform, methylene chloride, cyclic ethers, dialkylethers having 1 to 4 carbon atoms in each of the alkyl groups, or N,N-dialkylamides of lower aliphatic carboxylic acids.

16. A process according to claim 1, wherein the 2,2,4-trichloro-4-formylbutyronitrile formed by the addition reaction of trichloroacetaldehyde with acrylonitrile is firstly isolated, and is subsequently cyclised in a second stage of the process.

17. A process according to claims 1 and 16, wherein trichloroacetaldehyde is reacted with acrylonitrile at a temperature of 70 to 140°C in an inert solvent, in the presence of 0.1 to 5 mol % of copper powder, copper bronze, copper(I)- and copper(II)chloride or -bromide or copper(I) iodide, or in the presence of a mixture of these substances, in a closed system; and the 2,2,4-trichloro-4-formylbutyronitrile obtained is cyclised at a temperature of between 80 and 200°C in an open system, in the presence of hydrogen chloride or in the presence of a substance which forms hydrogen chloride under the reaction conditions, to give 2,3,5-trichloropyridine.

18. A process according to claim 1, wherein trichloroacetaldehyde and acrylonitrile are reacted at a temperature of between 70 and 220°C in the

presence of a catalyst, without isolation of the intermediately formed 2,2,4-trichloro-4-formylbutyronitrile, directly to 2,3,5-trichloropyridine.

19. A process according to claims 1 and 18, wherein the reaction of trichloroacetaldehyde and acrylonitrile is performed in a closed system at a pressure corresponding to the respective reaction temperature applied.

20. A process according to claims 1, 18 and 19, wherein the reaction of trichloroacetaldehyde and acrylonitrile is performed in the presence of an alkanecarboxylic acid nitrile having 2 to 5 carbon atoms or of a 3-alkoxypropionitrile having 1 to 2 carbon atoms in the alkyl group, as the solvent.

21. A process according to claims 1 and 18 to 20, wherein trichloroacetaldehyde and acrylonitrile are reacted in acetonitrile, butyronitrile or 3-methoxypropionitrile as the solvent, in the presence of 0.1 to 5 mol % of copper powder, copper bronze, copper(I)- or copper(II)chloride or -bromide or copper(I)iodide, or in the presence of a mixture of these substances, at 130 to 200° C, in a closed system at a pressure corresponding to the particular reaction temperature applied directly to 2,3,5-trichloropyridine.

22. 2,4,4-Trichloro-4-formylbutyronitrile.

23. A process for producing 2,4,4-trichloro-4-formylbutyronitrile, wherein trichloroacetaldehyde is caused to undergo, in the presence of a catalyst, an addition reaction with acrylonitrile.

24. A process according to claim 23, wherein the addition reaction of trichloroacetaldehyde with acrylonitrile is performed in the presence of an organic solvent at a temperature of 70 to 140° C.

25. A process according to claims 23 and 24, wherein the addition reaction of trichloroacetaldehyde with acrylonitrile is performed in the presence of excess acrylonitrile as the solvent.

26. A process according to claim 23, wherein the addition reaction of trichloroacetaldehyde with acrylonitrile is performed in the presence of a metal of the main group VIII or of the subgroups VIa, VIIa, Ib or IIb of the periodic system, of an oxide or of a salt of a metal of this type as the catalyst.

wherein the catalyst used for the addition reaction of trichloroacetaldehyde with acrylonitrile is iron(II)chloride, iron(III)chloride, iron powder, ruthenium(III)chloride, ruthenium(II)dichlorotristriphenylphosphine, copper powder, copper bronze, copper(I)chloride, copper(II)chloride, copper(I) bromide, copper(II)bromide, copper(II)acetate, copper(II)acetylacetonate, copper(II)benzylacetonate, copper(II)sulfate, copper(II)nitrate, copper(I)cyanide or copper(I)iodide.

28. A process according to claims 23 and 26, wherein the catalyst used for the addition reaction of trichloroacetaldehyde with acrylonitrile is copper powder, copper bronze, copper(I)chloride, copper(II)chloride, copper(I)bromide, copper(II)bromide or copper(I)iodide, or a mixture thereof.

29. A process according to claims 23 and 26 to 28, wherein the catalyst for the addition reaction of trichloroacetaldehyde with acrylonitrile is used in amounts of 0.01 to 10 mol %, preferably 0.1 to 5 mol %, relative to the acrylonitrile.

**Revendications**

1. Procédé de préparation de 2,3,5-trichloropyridine, caractérisé en ce qu'on fixe du trichloracétaldéhyde en présence d'un catalyseur sur de l'acrylonitrile et en ce qu'on cyclise le 2,4,4-trichloro-4-formylbutyronitrile de formule I

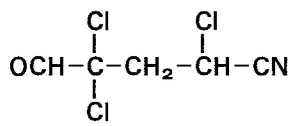

(I)

formé avec départ d'eau pour donner la 2,3,5-trichloropyridine.

2. Procédé selon la revendication 1, caractérisé en ce qu'on effectue la fixation de trichloroacétaldéhyde sur l'acrylonitrile à une température de 70 à 140° C.

3. Procédé selon la revendication 1, caractérisé en ce qu'on effectue la fixation de trichloroacétaldéhyde sur l'acrylonitrile dans un système fermé à une température de 70 à 140° C et à une pression correspondant à la température réactionnelle utilisée.

4. Procédé selon la revendication 1, caractérisé en ce qu'on utilise comme catalyseur pour la fixation du trichloroacétaldéhyde sur l'acrylonitrile un métal du groupe principal (VIII) ou des groupes secondaires VIa, VIIa, Ib et IIb, un oxyde d'un tel métal, un sel d'un tel métal ou un composé complexe d'un tel métal.

5. Procédé selon la revendication 1, caractérisé en ce qu'on utilise comme catalyseur pour la fixation de trichloracétaldéhyde sur l'acrylonitrile des sels et complexes de fer (II) et de fer (III), de la poudre de fer, du chlorure de ruthénium (III), de la ruthénium (II)-dichlorotristriphénylphosphine, de la poudre de cuivre, un mélange pulvérulent de cuivre et de bronze, des sels et complexes de cuivre (I) et de cuivre (II).

6. Procédé selon les revendications 1 et 5, caractérisé en ce qu'on utilise comme catalyseur pour la fixation de trichloracétaldéhyde sur l'acrylonitrile de la poudre de cuivre, un mélange pulvérulent de cuivre et de bronze, du chlorure et du bromure de cuivre (I) et de cuivre (II), de l'iodure de cuivre (I), ainsi que leurs mélanges.

7. Procédé selon les revendications 1 et 4 à 6, caractérisé en ce qu'on utilise le catalyseur pour la fixation de trichloracétaldéhyde sur l'acrylonitrile à des quantités d'environ 0,01 à 10% molaire, de préférence 0,1 à 5% molaire, par rapport à l'acrylonitrile.

8. Procédé selon la revendication 1, caractérisé en ce qu'on effectue la fixation de trichloraldéhyde sur l'acrylonitrile en présence d'un solvant organique inerte.

9. Procédé selon les revendications 1 et 8, caractérisé en ce qu'on effectue la fixation de trichloracétaldéhyde sur l'acrylonitrile dans un nitrile

d'acide alcanecarboxylique ayant de 2 à 5 atomes de carbone, un 3-alcoxypropionitrile ayant de 1 à 2 atomes de carbone dans le groupe alcoyle ou dans un excès d'acrylonitrile comme solvant.

10. Procédé selon la revendication 1, caractérisé en ce qu'on effectue la cyclisation du 2,4,4-trichloro-4-formylbutyronitrile formé par fixation de trichloroacétaldéhyde sur l'acrylonitrile à une température comprise entre 0 et 220°C, de préférence entre environ 80 et 200°C.

11. Procédé selon la revendication 1, caractérisé en ce qu'on effectue la cyclisation à une température comprise entre 80 et 200°C dans un système ouvert en présence d'acide chlorhydrique ou en présence de substances qui forment de l'acide chlorhydrique dans les conditions de la réaction.

12. Procédé selon les revendications 1 et 11, caractérisé en ce qu'on effectue la cyclisation du 2,4,4-trichloro-4-butyronitrile en présence d'acide bromhydrique et, en particulier, en présence d'acide chlorhydrique.

13. Procédé selon la revendication 1, caractérisé en ce qu'on effectue la cyclisation du 2,4,4-trichloro-4-formylbutyronitrile en l'absence de solvant, en chauffant en phase liquide ou en phase gazeuse.

14. Procédé selon la revendication 1, caractérisé en ce qu'on effectue la cyclisation du 2,4,4-trichloro-4-formylbutyronitrile en présence d'un solvant organique.

15. Procédé selon les revendication 1 et 14, caractérisé en ce qu'on effectue la cyclisation du 2,4,4-trichloro-4-formylbutyronitrile en présence de chloroforme, de chlorure de méthylène, d'éthers cycliques, de dialcoyléthers avec de 1 à 4 atomes de carbone dans les groupes alcoyle ou de N,N-dialcoylamide d'acides carboxyliques aliphatiques inférieurs.

16. Procédé selon la revendication 1, caractérisé en ce qu'on isole d'abord le 2,2,4-trichloro-4-formylbutyronitrile formé par fixation de trichloracétaldéhyde sur l'acrylonitrile, puis qu'on le cyclise dans une seconde étape du procédé.

17. Procédé selon les revendications 1 et 16, caractérisé en ce qu'on fait réagir le trichloracétaldéhyde à une température de 70 à 140°C dans un solvant inerte en présence de 0,1 à 5% molaire de poudre de cuivre, de mélange pulvérulent de cuivre et de bronze, de chlorure ou de bromure de cuivre (I) et de cuivre (II) ou d'iodure de cuivre (I) ou en présence d'un mélange de ces substances dans un système fermé avec l'acrylonitrile et en ce qu'on cyclise le 2,2,4-trichloro-4-formylbutyronitrile obtenu à une température comprise entre 80 et 220°C dans un système ouvert, en présence d'acide chlorhydrique ou d'une substance qui forme de l'acide chlorhydrique dans les conditions de la réaction, pour donner la 2,3,5-trichloropyridine.

18. Procédé selon la revendication 1, caractérisé en ce qu'on fait réagir du trichloracétaldéhyde et de l'acrylonitrile à une température comprise entre 70 et 220°C en présence d'un catalyseur sans isoler le 2,2,4-trichloro-4-formylbutyronitrile intermédiaire formé, pour donner directement la 2,3,5-trichloropyridine.

19. Procédé selon les revendications 1 et 18, caractérisé en ce qu'on effectue la réaction de trichloracétaldéhyde et d'acrylonitrile dans un système fermé à une température correspondant à la température réactionnelle utilisée.

20. Procédé selon les revendications 1, 18 et 19, caractérisé en ce qu'on effectue la réaction de trichloracétaldéhyde et d'acrylonitrile en présence d'un nitrile d'acide alcanecarboxylique avec de 2 à 5 atomes de carbone ou d'un 3-alcoxypropionitrile avec de 1 à 2 atomes de carbone dans le groupe alcoyle comme solvant.

21. Procédé selon les revendications 1 et 18 à 20, caractérisé en ce qu'on fait réagir du trichloracétaldéhyde et de l'acrylonitrile dans l'acétonitrile, le butyronitrile ou le 3-méthoxypropionitrile comme solvant en présence de 0,1 à 5% molaire de poudre de cuivre, de mélange pulvérulent de cuivre et de bronze, de chlorure ou de bromure de cuivre (I) ou de cuivre (II) ou d'iodure de cuivre (I), ou d'un mélange de ces substances à 130 à 200°C dans un système fermé à une pression correspondant à la température réactionnelle utilisée à chaque fois, pour donner directement la 2,3,5-trichloropyridine.

22. Le 2,4,4-trichloro-4-formylbutyronitrile.

23. Procédé de préparation de 2,4,4-trichloro-4-formylbutyronitrile, caractérisé en ce qu'on fixe du trichloracétaldéhyde en présence d'un catalyseur sur l'acrylonitrile.

24. Procédé selon la revendication 23, caractérisé en ce qu'on procède à la fixation de trichloroacétaldéhyde sur l'acrylonitrile en présence d'un solvant organique à une température de 70 à 140°C.

25. Procédé selon les revendications 23 et 24, caractérisé en ce qu'on effectue la fixation de trichloracétaldéhyde sur l'acrylonitrile en présence d'un excès d'acrylonitrile comme solvant.

26. Procédé selon la revendication 23, caractérisé en ce qu'on effectue la fixation de trichloracétaldéhyde sur l'acrylonitrile en présence d'un métal du groupe principal VIII ou des groupes secondaires VIa, VIIa, Ib ou IIb de la classification périodique des éléments, d'un oxyde ou d'un sel d'un tel métal comme catalyseur.

27. Procédé selon les revendications 23 et 26, caractérisé en ce qu'on utilise comme catalyseur pour la fixation de trichloracétaldéhyde sur l'acrylonitrile le chlorure de fer (II), le chlorure de fer (III), la poudre de fer, le chlorure de ruthénium (III), la ruthénium (II)-dichlorotristriphénylphosphine, la poudre de cuivre, un mélange pulvérulent de cuivre et de bronze, le chlorure de cuivre (I), le chlorure de cuivre (II), le bromure de cuivre (I), le bromure de cuivre (II), l'acétate de cuivre (II), l'acétylacétonate de cuivre (II), le benzylacétonate de cuivre (II), le sulfate de cuivre (II), le nitrate de cuivre (II), le cyanure de cuivre (I) ou l'iodure de cuivre (I).

28. Procédé selon les revendications 23 et 26, caractérisé en ce qu'on utilise comme catalyseur

pour la fixation de trichloracétaldéhyde sur l'acrylonitrile de la poudre de cuivre, un bronze cuivré, le chlorure de cuivre (I), le chlorure de cuivre (II), le bromure de cuivre (I), le bromure de cuivre (II) ou l'iodure de cuivre (I), ou leurs mélanges.

29. Procédé selon les revendications 23 et 26 à 28, caractérisé en ce qu'on utilise des catalyseurs pour la fixation de trichloracétaldéhyde sur l'acrylonitrile à des quantités de 0,01 à 10% molaire, de préférence 0,1 à 5% molaire, par rapport à l'acrylonitrile.